# EUROPÄISCHE PATENTSCHRIFT

(11) **EP 1 007 109 B1**
(45) Veröffentlichungstag und Bekanntmachung des Hinweises auf die Patenterteilung: **26.03.2003**
(21) Anmeldenummer: 98941134.3
(22) Anmeldetag: 26.08.1998
(51) Int. Cl.: A61L 24/00

(54) **GEWEBEKLEBER AUF BASIS VON FIBRINOGEN**
FIBRINOGEN-BASED TISSUE ADHESIVE
ADHESIF TISSULAIRE A BASE DE FIBRINOGENE

(30) Priorität: 28.08.1997 AT 144997
(43) Veröffentlichungstag der Anmeldung: 14.06.2000
(73) Patentinhaber: Baxter Aktiengesellschaft, 1221 Wien (AT)
(72) Erfinder: REDL, Heinz, A-1060 Wien (AT); SCHLAG, Günther, A-1190 Wien (AT); EIBL, Johann, A-1180 Wien (AT)
(74) Vertreter: Alge, Daniel, Mag. Dr. rer.nat.
(86) Internationale Anmeldenummer: AT9800202
(87) Internationale Veröffentlichungsnummer: WO99011301

(56) Entgegenhaltungen:
- EP-A- 0 253 198
- AT-B- 374 367
- SIMON D I ET AL: "FIBRIN(OGEN) IS INTERNALIZED AND DEGRADED BY ACTIVATED HUMAN MONOCYTOID CELLS VIA MAC-1 (CD11B/CD18): A NONPLASMIN FIBRINOLYTIC PATHWAY" BLOOD, Bd. 82, Nr. 8, 15. Oktober 1993, Seiten 2414-2422, XP002059464 in der Anmeldung erwähnt
- PLESCIA J ET AL.: "ACTIVATION OF MAC-1 (CD11b/CD18)-BOUND FACTOR X BY RELEASED CATHEPSIN G DEFINES AN ALTERNATIVE PATHWAY OF LEUCOCYTE INITIATION OF COAGULATION" BIOCHEMICAL JOURNAL, Bd. 319, Nr. 3, 1996, Seiten 873-879, XP002089309

## Beschreibung

Die Erfindung betrifft einen Gewebekleber auf Basis von Fibrinogen.

Gewebekleber auf Basis von Fibrinogen, die auch als Fibrinkleber bezeichnet werden, imitieren bei ihrer Klebewirkung die letzte Phase der Blutgerinnung. Dabei wird Fibrinogen durch die Einwirkung von Thrombin, welches beim Klebevorgang meist der Fibrinogenlösung zugesetzt wird, aber auch in jeder Wunde vorhanden ist, in Fibrinmonomere gespalten. Die Fibrinmonomere lagern sich spontan zu geordneten faserförmigen Strukturen zusammen, die man als Fibrin bezeichnet. Dieses Fibrinmonomeraggregat wird dann unter Einwirkung von Faktor XIIIa durch kovalente Quervernetzungen weiter stabilisiert. Dabei bilden sich zwischen spezifischen Glutamin- und Lysinseitenketten der Fibrinmonomere in einer Transamidierungsreaktion Peptidbindungen aus. Der Faktor XIIIa, welcher ebenfalls durch Thrombin aus inaktivem Faktor XIII gespalten wird, ist eine aktive Transamidase und wird aufgrund seiner Wirkung auch als "fibrinstabilisierender Faktor" bezeichnet.

Obwohl mit der Anwendung eines Gewebeklebers prinzipiell dieselben Prozesse wie bei der "natürlichen" Blutgerinnung ablaufen, so sind bei einem Gewebekleber die daran beteiligten Komponenten und Faktoren doch um ein Vielfaches konzentrierter als im Blut. Dadurch läuft die Blutgerinnung auch sehr viel schneller ab und die erzielte Gewebeklebung oder das gebildete Blutgerinnsel sind sehr viel sicherer und auch stabiler.

Voraussetzung für den Durchbruch der Fibrinkleber Ende der 70er Jahre waren die Fortschritte in der Fraktionierung und Reinigung von Blutgerinnungsfaktoren. Erst dadurch war es möglich, die natürlichen Gerinnungsfaktoren so rein und konzentriert herzustellen, wie es für eine effiziente Gewebeklebung notwendig ist. Die ersten kommerziell erhältlichen Gewebekleber gelangten Ende der 70er Jahre auf den Markt und haben sich seither in einer Vielzahl von Einsatzmöglichkeiten bewährt; v.a. in den Bereichen, in denen es mit herkömmlicher chirurgischer Technik immer wieder zu großen Problemen kam, z.B. bei starken Blutungen, bei Nervenklebungen oder bei Rissen innerer Organe wie Leber und Milz.

Ein weiterer Vorteil eines Fibrinklebers im Gegensatz zu Nähen mit Nadel und Faden besteht darin, daß das zu behandelnde Gewebe oder das Organ nicht durch einen Nähvorgang noch zusätzlich geschädigt wird, weshalb es bei der Anwendung von Gewebeklebern auf Basis von Fibrinogen viel weniger Komplikationen und unauffälligere Narben gibt als an herkömmlichen chirurgischen Nähten. Neben der optimalen Klebewirkung, welche eine hohe Belastbarkeit und eine hohe innere Festigkeit der Klebungen sowie gute Haftfähigkeit des Klebers an den Wund- bzw. Gewebsflächen beinhaltet, sind auch die der unmittelbaren Klebung folgenden Prozesse für die Optimierung von Gewebeklebern wesentlich (s. AT-B-359 652 und 359 653). Dazu gehören die Steuerung und Kontrolle der Haltbarkeit der Klebungen im Körper sowie die Resorbierbarkeit und die wundheilungsfördernden Eigenschaften des Klebstoffes.

Daher ist für einen Gewebekleber nicht nur die schnelle und sichere Klebewirkung von entscheidender Bedeutung, sondern auch, daß sich die entstandene Klebung bzw. das entstandene Blutgerinnsel innerhalb einer bestimmbaren Zeitspanne im Körper wieder auflöst und die Wunde in Folge der vollkommenen Resorption des entstandenen Gerinnsels wieder vollkommen ausheilt.

Dabei ist es notwendig, den (körpereigenen) Prozeß der Auflösung des entstandenen Blutgerinnsels, die Fibrinolyse, ebenfalls durch die Optimierung des Gewebeklebers zu steuern.

Bei der Fibrinolyse wird das im entstandenen Blutgerinnsel vorhandene Fibrin abgebaut bzw. entfernt und dadurch das Blutgerinnsel aufgelöst. Dabei wird zunächst unter dem Einfluß intrinsischer oder extrinsischer Plasminogen-Aktivatoren, wie Blutgerinnungsfaktoren XI und XII, Präkallikrein, Urokinase oder t-PA, aus dem inaktiven Plasminogen das fibrinolytisch wirksame Plasmin gebildet, welches neben Fibrin auch Fibrinogen und die Blutgerinnungsfaktoren V und VIII spaltet.

Da die körpereigenen Fibrinolyse-Prozesse meist unmittelbar nach Entstehen eines Gerinnsels einsetzen und somit die Gefahr beteht, daß eine entstandene Gewebeklebung nicht fest genug haften bleibt bzw. ein entstandenes Gerinnsel vorzeitig destabilisiert wird, sieht man bei der Gewebeklebung in der Regel den Zusatz eines Plasmininhibitors oder eines Plasminogenaktivator-Inhibitors vor, um die Wirkung von Plasmin direkt oder indirekt zu hemmen und so, v.a. in der Anfangsphase der Klebung, diese vor vorzeitiger Fibrinolyse zu bewahren. Mit der Konzentration des Inhibitors lassen sich auch die Auflösezeiten (Lysezeiten) des entstandenen Gerinnsels bzw. der Klebung gezielt steuern. Je mehr Inhibitor vorgesehen wird, desto stabiler ist das Gerinnsel gegenüber Fibrinolyse, desto länger bleibt dieses Gerinnsel also stabil und desto länger dauert es auch, bis der Kleber vollständig resorbiert wird.

Es gilt also bei Einsatz des Fibrinolyseinhibitors einen optimalen Mittelweg zwischen dem Unterbinden der frühen Fibrinolyse und einem möglichst schnellen Wundheilungsprozeß zu finden.

Als Plasmininhibitor wird in den kommerziell erhältlichen Gewebeklebern Aprotinin verwendet, der auch als boviner basischer pankreatischer Trypsin-Inhibitor bezeichnet wird. Aprotinin ist ein polyvalenter Proteinasen-(Kallikrein)-Inhibitor und hemmt die Gerinnungsfaktoren XIIa, XIa, VIIIa sowie v.a. Plasmin und Plasminaktivatoren, aber auch Trypsin, Chymotrypsin und Kallikrein.

Aprotinin wurde früher hauptsächlich aus Rindern hergestellt. Bedingt durch die Problematik der Verwendung von bovinem Material in Arzneimitteln, die zur Behandlung von Menschen eingesetzt werden, wird aber immer häufiger rekombinant hergestelltes Aprotinin verwendet.

Aprotinin wird bei der Gewebeklebung in der Regel in einer Menge von 20-3000 Kallikrein-Inaktivator-Einheiten (KIE)/ml Gewebekleber eingesetzt, wobei die optimale Konzentration von der fibrinolytischen Aktivität des jeweiligen Gewebes abhängt.

Es hat sich aber gezeigt, daß v.a. in Geweben mit hoher fibrinolytischer Aktivität die Fibrinolyse-inhibitorische Wirkung von Aprotinin trotz des Einsatzes hoher Aprotinin-Konzentrationen nur sehr begrenzt steuerbar ist und es daher zu unerwünschten, frühzeitigen Lyseprozessen kommen kann.

Aufgabe der vorliegenden Erfindung ist es daher, einen Gewebekleber zur Verfügung zu stellen, mit dem die Nachteile im Stand der Technik überwunden werden, und womit auch v.a. bei Gewebeklebung in Wunden mit hoher Plasminaktivität ein zufriedenstellender und verläßlicher Schutz gegen frühzeitige Fibrinolyse gewährleistet wird, wobei die Qualität der Klebung nicht beeinträchtigt werden darf.

Diese Aufgabe wird erfindungsgemäß gelöst durch einen Gewebekleber auf Basis von Fibrinogen, welcher sich dadurch auszeichnet, daß er einen zugesetzten Elastase-Inhibitor enthält. Es hat sich nämlich überraschenderweise gezeigt, daß der Fibrinolyseprozeß nicht nur durch Inhibierung von Plasmin bzw. die Inhibierung der Aktivierung von Plasminogen zu Plasmin verhindert werden kann, sondern auch durch Elastase-Inhibitoren bzw. durch Inhibitoren, deren Fibrinolyse-inhibitorische Wirkung überwiegend auf einem nicht-Plasmin-Fibrinolysemechanismus beruht. Für die Zwecke der vorliegenden Erfindung werden solche Non-Plasminogen-Fibrinolyse-Inhibitoren der Einfachheit halber dem Begriff "Elastase-Inhibitor" unterstellt. Es wurde zwar spekuliert, daß es neben der plasminvermittelten Fibrinolyse noch weitere Fibrinolyseprozesse geben könnte, die nicht auf Plasmin beruhen (etwa ein Prozeß, der lysosomal abläuft; s. Simon et al., BLOOD 82(8) (1993), Seiten 2414-2422), und durch Aprotinin nicht wesentlich inhibiert werden können, es zeigte sich aber auch, daß dieser "non-plasmin fibrinolytic pathway" auch nicht durch spezifische Elastase-inhibierende Peptide, wie N-Methoxy-Succinyl-L-Alanyl-L-Prolyl-L-Valanylchlormethylketon (AAPVCK) inhibiert werden konnte (s. Simon et al.). Umso überraschender war es, daß im Zuge der vorliegenden Erfindung herausgefunden werden konnte, daß Inhibitoren, die keine (wesentlichen) Plasmin- oder Plasminogen-Aktivator-inhibierende Wirkungen aufweisen, also die erfindungsgemäßen Elastase-Inhibitoren bei Fibrinklebern sowohl in vitro als auch in vivo einen sehr gut kontrollierbaren Lyseprozeß des gebildeten Gerinnsels gewährleisten können. Dies stellte sich als besonders vorteilhaft in Geweben mit erhöhter fibrinolytischer Aktivität heraus, in denen sie bereits in moderaten Konzentrationen eine frühzeitige Lyse verhindern können.

Die vorzeitige Lyse spielt bei Geweben mit hoher fibrinolytischer Aktivität v.a. auch eine Rolle innerhalb der ersten Zeit nach der Klebung, da es bei vorzeitiger Lyse zu einem (teilweise) Ablösen der Klebung und somit zu einem erneuten Blutungsprozeß ("Rebleeding") kommen kann.

Es zeigte sich weiters, daß der erfindungsgemäß im Gewebekleber zu verwendende Elastase-Inhibitor die fibrinolytische Wirkung nicht nur in Kombination mit herkömmlichen, auf Plasmin wirkenden Inhibitoren aufwies, sondern daß auch die gesamte Fibrinolyseinhibierung vom Elastase-Inhibitor allein gewährleistet werden kann. Eine besondere Ausführungsform der vorliegenden Erfindung besteht daher darin, daß der Gewebekleber außer Fibrinogen dem Elastase-Inhibitor und gegebenenfalls Faktor XIII keine weiteren aktiven Komponenten enthält.

Die Fibrinogenkonzentration beim vorliegenden Kleber entspricht der bekannter Gewebekleber und sollte in der Regel zumindest über 50 mg, insbesondere über 70-80 mg Fibrinogen/ml liegen, also mindestens etwa das 20-fache der Fibrinogenkonzentration in Blut (2-4 mg/ml) betragen. Vorzugsweise liegt das Fibrinogen in gegenüber Kryopräzipitat weiter gereinigter Form vor.

Bevorzugte Elastase-Inhibitoren sind im Rahmen der vorliegenden Erfindung ausgewählt aus der Gruppe Eglin, Elastase-α₁-Proteinase-Inhibitor, α₁-Antiprotease, Elafin, Leukozytenprotease-Inhibitor, insbesondere eine Leukozytenfraktion, vorzugsweise eine von Granulozyten abgeleitete Fraktion, oder humaner sekretorischer Leukoprotease-Inhibitor, oder Mischungen davon. Als Leukozytenfraktion kann beispielsweise ein Zell-Lysat, insbesondere eines von humanen Zellen abgeleitetes, verwendet werden. Weitere Elastase- oder andere nicht auf Plasmin wirkende Fibrinolyse-Inhibitoren können vom Fachmann in einfacher Weise durch die in den Beispielen offenbarten Testsysteme im Hinblick auf ihre Eignung im erfindungsgemäßen Gewebekleber untersucht werden oder unter Anwendung der aus dem Stand der Technik bekannten Elastase-Hemmtests. Zu den bevorzugten Elastase-Inhibitoren zählen auch verschiedene Derivate der erfindungsgemäßen Elastase-Inhibitoren, beispielsweise Fragmente oder chemisch oder durch (rekombinantes) Protein-Design modifizierte Formen dieser Inhibitoren, wobei diese Derivate jedoch selbstverständlich immer die qualitative Elastase-Inhibitor-Eigenschaft des Basisinhibitors aufweisen müssen.

Bevorzugterweise besteht der erfindungsgemäße Gewebekleber ausschließlich aus menschlichen Proteinen, wobei unter "menschlichen Proteinen" auch die rekombinant hergestellten Humanproteine zu verstehen sind. Vorzugsweise werden daher die im Gewebekleber verwendeten Proteine entweder aus Blut, Plasma, Kryopräzipitat oder aus einer rekombinanten Zellkultur hergestellt.

Ein besonders bevorzugter Gewebekleber zeichnet sich dadurch aus, daß er ausschließlich aus menschlichem Blut oder Plasmaproteinen zusammengesetzt ist.

Das Mengenverhältnis von Elastase-Inhibitor zu mg Fibrinogen beträgt vorzugsweise 1:100 bis 1:150 000, vorzugsweise 1:500 bis 1:110 000. In Einheiten Inhibitor zu g Fibrinogen ausgedrückt werden dem Gewebekleber vorzugsweise wenigstens 10⁻⁶ E/g Fibrinogen zugesetzt. Besonders bevorzugt ist ein Bereich zwischen 10⁻³ und 10 E/g Fibrinogen. Die Menge an zugesetztem Inhibitor in dem erfindungsgemäßen Gewebekleber, welcher Inhibitor auch natürlicherweise in Blut bzw. Plasma vorhanden sein kann, ist vorzugsweise mindestens 20x, insbesondere mindestens 50x höher, als dessen physiologische Konzentration in Blut bzw. Plasma. Der erfindungsgemäße Gewebekleber kann beispielsweise folgendermaßen zusammengesetzt sein: 75-115 mg/ml clottierbares Protein, davon 50-110 mg/ml, vorzugsweise 70-110 mg/ml Fibrinogen; gegebenenfalls 1-50, vorzugsweise 10-50 IE Faktor XIII/ml. Als Inhibitor kann beispielsweise Eglin in einer Menge zwischen 1-100 µg/ml oder α₁-Antiprotease mit 0,01-1 E/ml zugesetzt werden. In der Regel ist es ausreichend, den Elastase-Inhibitor in einer Menge zuzusetzen, welche der Fibrinolyse-inhibierenden Wirkung von Aprotinin in bekannten Gewebeklebern entspricht.

Der erfindungsgemäße Kleber kann je nach Zweck der Klebung Plasminogen enthalten oder plasminogenfrei sein. Wenn Plasminogen enthalten ist, sollte es in einer Menge von zumindest 0,0001 mg/mg Fibrinogen, vorzugsweise mehr als 0,001, insbesondere mehr als 0,01, enthalten sein. Mit der Anwesenheit von Plasminogen im Gewebekleber ist, aufgrund dessen Aktivierung zu Plasmin, ebenfalls eine Möglichkeit gegeben, die Fibrinolyse-Eigenschaften des Gewebeklebers noch deutlicher zu definieren.

Andererseits enthält der Gewebekleber in einer weiteren bevorzugten Ausführungsform überhaupt kein Plasminogen bzw. nur in geringer Menge.

Wie erwähnt, reicht die Anwesenheit des Elastase-Inhibitors als einzigem Fibrinolyse-Inhibitor in einem Gewebekleber überraschenderweise für die Funktionalität des erfindungsgemäßen Klebers aus. Bevorzugterweise wird allerdings neben dem Elastase-Inhibitor auch ein Plasmin-Inhibitor oder ein Plasmin-Aktivator-Inhibitor verwendet, was ebenfalls zur besseren Kontrolle der Lyse der Resorption und somit der Wundheilung beiträgt. Bevorzugte Plasmin-Inhibitoren oder Plasminogen-Aktivator-Inhibitoren sind v.a. Aprotinin, aber auch α₂-Makroglobulin, α₁-Antitrypsin. ε-Amino-Capronsäure, Tranexamsäure oder Mischungen dieser Substanzen. Obwohl vereinzelt Autoren auch z.B. α₁-Antitrypsin gewisse Wirkungen auf Elastase zugeschrieben haben, werden die hier erwähnten Substanzen als Plasmin- bzw. Plasminogen-Aktivatoren angesehen, da dies die primäre Aktivität ist, die diese Substanzen auf dem vorliegenden Gebiet aufweisen. Dies gilt daher selbstverständlich auch für die Zwecke der vorliegenden Erfindung. Des weiteren können auch anti-adhäsive Zusätze, z.B. Hyaluronsäure, enthalten sein.

Eine weitere bevorzugte Ausführungsform des erfindungsgemäßen Gewebeklebers besteht darin, ein Antibiotikum im Kleber vorzusehen, wie bereits in der AT-B-369 990 vorgeschlagen worden ist. Besonders bevorzugte Antibiotika sind dabei ausgewählt aus der Gruppe Aminoglycoside, Betalactame, Polypeptide, Fosfomycin, Tetracycline oder Mischungen davon. In einer weiteren bevorzugten Ausführungsform liegt das Antibiotikum in Form eines schwerlöslichen Derivats vor.

Bevorzugterweise wird im erfindungsgemäßen Gewebekleber auch Faktor XIII vorgesehen, so daß die innere Festigkeit des Clots und die Stärke und Haltbarkeit der Klebung positiv beeinflußt werden. Faktor XIII wird dafür vorzugsweise in einer Menge von 1-50 Einheiten/ml, vorzugsweise um 10 E/ml, verwendet. In Bezug auf Fibrinogen liegt Faktor XIII vorzugsweise in einer Mindestkonzentration von 0,001 E/mg Fibrinogen, insbesondere zumindest 0,1 E/mg Fibrinogen vor. Je nach Klebungsart oder Gewebetyp kann aber die optimale Faktor XIII-Konzentration von jedem Fachmann leicht optimiert werden. Ist ein Antibiotikum im Gewebekleber vorhanden, so empfiehlt es sich grundsätzlich, etwas mehr Faktor XIII vorzusehen (vgl. AT-B-369 990).

Bevorzugterweise ist der erfindungsgemäße Gewebekleber frei von kininogenen Proteinen (wie z.B. Kallikrein, etc.), wodurch störende Nebenreaktionen von vornherein unterbunden werden können.

Gemäß einer bevorzugten Ausführungsform wird der erfindungsgemäße Gewebekleber in Kombination mit einer festen Oberfläche als Vlies vorgelegt, womit v.a. für großflächige Wunden ein optimaler Wundverschluß und eine optimale Abdeckung erzielt wird. Beispiele derartiger Vliese sind in der AT-B 374 367 genannt. Die feste Oberfläche des Vlieses ist daher bevorzugterweise eine Kollagen-, Gelatine- oder eine Polysaccharid-Oberfläche, wobei durchaus weitere medizinisch geeignete Oberflächen, die auch gegebenenfalls für den speziellen Verwendungszweck imprägniert sein können, verwendet werden können.

Es hat sich gezeigt, daß erfindungsgemäß mit dem Gewebekleber, enthaltend einen Elastase-Inhibitor, sogar in einem Milieu mit hoher fibrinolytischer Aktivität für einen Zeitraum von wenigstens 10 Stunden, vorzugsweise 15 Stunden, eine Lysebeständigkeit erzielt werden kann. Lysebeständig bedeutet gemäß der vorliegenden Erfindung, daß ein entsprechender Fibrinclot innerhalb eines bestimmten Zeitraumes nicht abgebaut wird, also bestehen bleibt. Die Bestimmung der Lysebeständigkeit erfolgt beispielsweise durch eine photometrische Messung in Abhängigkeit von der Zeit. Ein bevorzugter Gewebekleber gemäß der vorliegenden Erfindung weist daher eine Lysebeständigkeit von wenigstens 10 Stunden, vorzugsweise wenigstens 15 Stunden, in einem Milieu hoher fibrinolytischer Aktivität auf. Unter "hoher fibrinolytischer Aktivität" wird beispielsweise eine Plasminaktivität verstanden, die über dem physiologischen Plasmin-Potential liegt. Das fibrinolytische Potential kann beispielsweise durch die Plasminogen-Konzentration ausgedrückt werden (siehe z.B. Henriksson et al., Thrombosis Research 16: 301-312; 1979) Diese Eigenschaft der Lysebeständigkeit kann von jedem Fachmann durch einen einfachen Test, wie in den Beispielen beschrieben, überprüft werden.

Bei der Applikation liegt der erfindungsgemäße Gewebekleber vorzugsweise in Lösung vor, zur Lagerung empfiehlt sich aber entweder das Tieffrieren der Lösung, so daß der erfindungsgemäße Gewebekleber in tiefgefrorener Form vorliegt, oder das Lyophilisieren des Klebers, also das Vorsehen in lyophilisierter Form. Unter "lyophilisierter Form" wird selbstverständlich nur eine durch Gefriertrocknung haltbar gemachte Gewebekleberpräparation verstanden, die bei anschließender Rekonstitution nahezu vollständig (d.h. zu zumindest 80%) innerhalb von wenigen Minuten bei 37°C rekonstituiert werden kann.

Der erfindungsgemäße Kleber liegt vorteilhafterweise in virusinaktivierter Form vor.

Diese Inaktivierungsbehandlung wird vorzugsweise mit einer Tensid- und/oder Hitzebehandlung gewährleistet, beispielsweise durch eine Hitzebehandlung in festem Zustand, insbesondere eine Dampfbehandlung gemäß der EP-0 159 311, oder der EP-0 519 901 oder der EP-0 674 531.

Weitere Behandlungen zur Inaktivierung von Viren umfassen auch die Behandlung mit chemischen oder chemisch/physikalischen Methoden, z.B. mit chaotropen Stoffen gemäß der WO94/13329, der DE 44 34 538 oder der EP-0 131 740 (Lösungsmittel) oder die Photoinaktivierung.

Die Nanofiltration stellt ebenfalls ein bevorzugtes Verfahren zur Abreicherung von Viren im Rahmen der vorliegenden Erfindung dar.

Die erfindungsgemäß zugesetzten Elastase-Inhibitoren können gemäß einer bevorzugten Ausführungsform auch rekombinanten Ursprungs sein.

Die vorliegende Erfindung betrifft weiters ein Gewebeklebersystem, welches als eine Komponente einen erfindungsgemäßen Gewebekleber, enthaltend einen Elastase-Inhibitor, umfaßt.

Das erfindungsgemäße Gewebeklebersystem enthält in der Regel als weitere Komponente eine Thrombin-Komponente, in welcher Thrombin entweder in flüssiger Form oder als rekonstituierbares Lyophilisat vorliegt, wobei die Thrombin-Komponente beim Klebeeinsatz je nach Anwendungsgebiet unterschiedlich konzentriert sein kann.

Ein Gewebeklebersystem, welches ebenfalls unter die vorliegende Erfindung fällt, zeichnet sich dadurch aus, daß es eine Fibrinogen-Komponente und eine davon getrennte Komponente umfaßt, die einen Elastase-Inhibitor enthält. In der Regel ist es jedoch günstig, die Fibrinolyse-Inhibitor-Komponente in der Fibrinogen-Komponente vorzusehen (s. AT-B-359 652 und 359 653). Durch geeignete Applikationsvorrichtungen kann aber die Inhibitor-Komponente auch getrennt von der Fibrinogen-Komponente zugeführt werden. Vorzugsweise enthält die Komponente, die einen Elastase-Inhibitor enthält, auch gleichzeitig Thrombin, wobei diese Komponente wiederum entweder als Lyophilisat oder als (ggf. tiefgefrorene) Lösung zur Verfügung gestellt werden kann.

Die erfindungsgemäßen Gewebeklebersysteme umfassen weiters geeignete Applikationsvorrichtungen für die Systemkomponente(n). Insbesondere haben sich dabei Doppelspritzensysteme, wie in den EP 0 037 393, EP 0 210 160 oder EP 0 292 472, oder aber Applikationsvorrichtungen wie in den EP 0 315 322 oder EP 0 669 100 beschrieben, bewährt. Mit diesen speziellen Applikationsvorrichtungen kann auch diejenige Ausführungsform, bei welcher der Inhibitor in der Thrombin-Komponente appliziert wird, verläßliche Klebeergebnisse liefern.

Der vorliegende Kleber ist für alle bisher bekannten Applikationsmöglichkeiten der Fibrinkleber geeignet. Er hat sich aber besonders bei der Klebung in Bereichen mit hoher fibrinolytischer Aktivität bewährt. Gegenstand der vorliegenden Erfindung ist daher auch die Verwendung des erfindungsgemäßen Gewebeklebers bzw. eines erfindungsgemäßen Gewebeklebersystems zur Herstellung einer Präparation bzw. einer Applikationsvorrichtung zur Anwendung in Bereichen mit hoher fibrinolytischer Aktivität, insbesondere in der Urologie. Gegenstand der vorliegenden Erfindung ist weiters ein Verfahren zur Anwendung eines erfindungsgemäßen Gewebeklebers oder eines erfindungsgemäßen Gewebeklebersystems in der Chirurgie in Bereichen mit hoher fibrinolytischer Aktivität, insbesondere in der Urologie.

Die Erfindung wird anhand der nachfolgenden Beispiele und der Zeichnungsfiguren, auf die sie jedoch nicht eingeschränkt werden soll, näher erläutert.

Es zeigen:
Fig. 1: die Abnahme der Extinktion entsprechend einer zunehmenden Lyse des Clots
   a) Fibrinkleber ohne Aprotinin
   b) Fibrinkleber mit Aprotinin (1000 U/ml)
   c) Fibrinkleber mit Alphal-PI (0,01 U/ml)
   d) Fibrinkleber mit Alphal-PI (0,001 U/ml)
   e) Fibrinkleber mit Alphal-PI (0,0001 U/ml);
Fig. 2: die Abnahme der Extinktion entsprechend einer zunehmenden Lyse des Clots
   a) Fibrinkleber mit Aprotinin (1000 U/ml)
   b) Fibrinkleber mit Eglin (1 µg/ml)
   c) Fibrinkleber ohne Aprotinin;
Fig. 3: das Ausmaß des "Rebleedings" ausgedrückt durch Gewichtszunahme von vorgewogenen Tupfen in hyperfibrinolytischem Milieu, welches durch Infusion von t-PA induziert wurde; und
Fig. 4: das Ausmaß des "Rebleedings" ausgedrückt durch Gewichtszunahme von vorgewogenen Tupfen in Milieu mit normaler fibrinolytischer Aktivität, also ohne t-PA Infusion.

### Beispiele:

### 1. In vitro-Test zur Untersuchung der Fibrinolyse-inhibierenden Wirkung des erfindungsgemäßen Gewebeklebers (derzeit nach Ansicht der Anmelderin der beste Weg zur Ausführung der Erfindung)

In diesem Beispiel wird die Blockade der Lyse eines Gewebekleberclots mittels Eglin oder α₁-Antiprotease gezeigt. Dabei wird der Gewebekleber STIM3 (IMMUNO AG, Wien, AT) (enthaltend 70 mg Fibrinogen/ml) in Wasser gelöst und anschließend mit einer 0,9 M NaCl-Lösung 1:6 verdünnt.

Diese Gewebekleberlösung wird mit einer in 40 mM CaCl₂ gelösten und anschließend mit einer 40 mM CaCl₂/0,9 M NaCl (1:5)-Lösung auf 0,1 E/ml verdünnte Thrombin-Lösung im Verhältnis 1:1 gemischt und auf eine Mikroplatte pipettiert, wobei 100 µl/well vorgesehen werden.

Verschiedene Konzentrationen der Inhibitoren wurden in jeweils 5 µl dem Gewebekleber zugesetzt (Eglin 1-100 µg/ml,α₁-Antiprotase 0,01-1 E/ml).

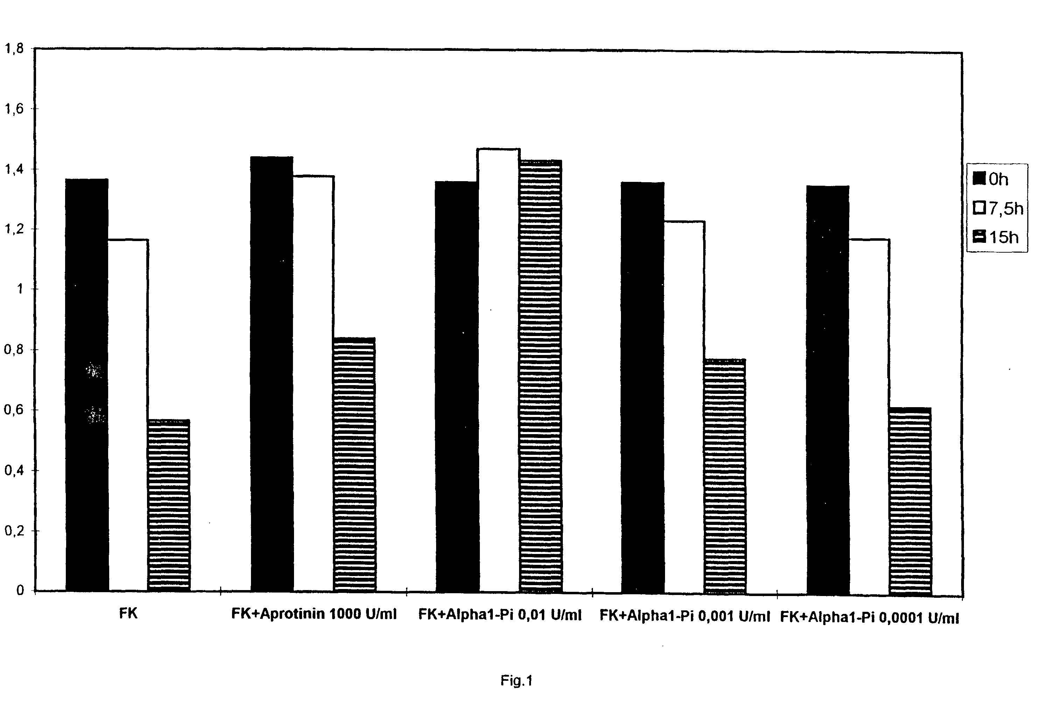

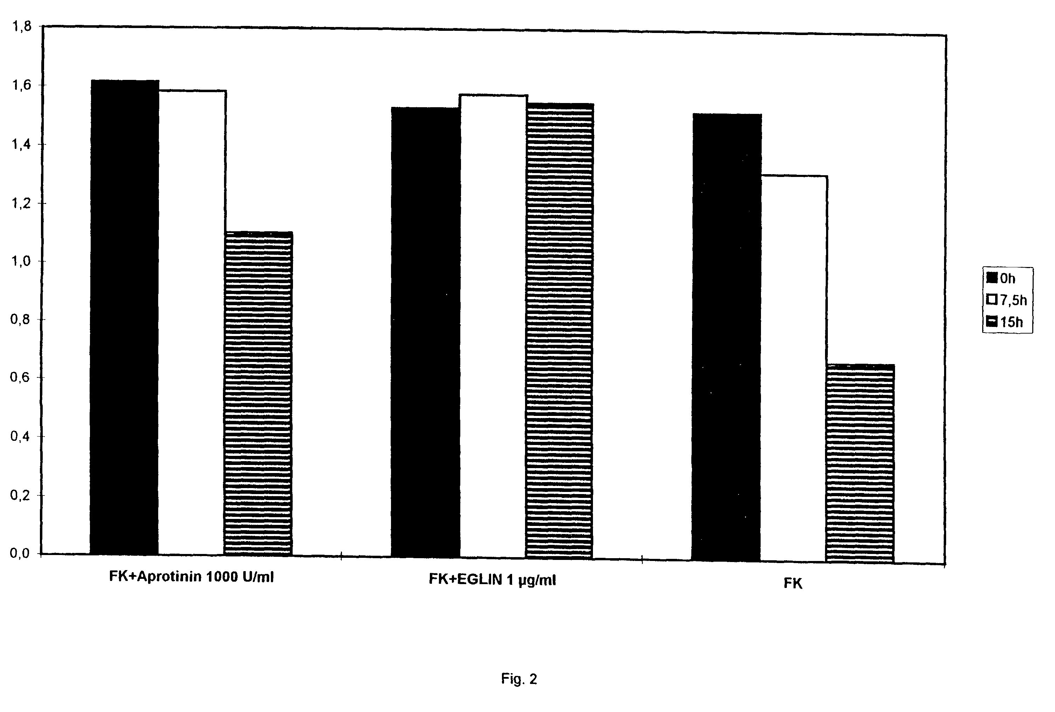

Zum Aushärten des Klebers wurde die Mikrotiterplatte ca. 1,5 Stunden bei 37°C inkubiert. Die entsprechenden Lysereagenzien (a: zellfreier Überstand aus Leukozytenhomogenat (3x Einfrieren/Auftauen) von 500 000 Leukozyten/µl; b: t-PA 2mg/ml als Positivkontrolle; NaCl 0,9% als Negativkontrolle) wurden dann auf die Gerinnsel pipettiert (100 µl/well). Anschließend wurden die Mikrotiterwells im Plattenphotometer bei 37°C bei einer Wellenlänge von 405 nm kinetisch über Nacht 60x900 s im Photometer SLT 340 ATTC gemessen. Die Ergebnisse sind in den Fig. 1 und 2 dargestellt, wobei die Abnahme der Extinktion der zunehmenden Lyse des Clots entspricht.

Es zeigte sich, daß sowohl mit ≥ 1 µg Eglin/ml als auch mit ≥ 0,01 E α₁-Antiprotease/ml es möglich ist, die im Versuch innerhalb von 15 Stunden ablaufende Lyse des Fibrinclots zu verhindern, was einerseits auf die zentrale Rolle von Leukozytenproteasen für den Abbau des Fibrinclots schließen läßt und die ausgezeichnete Wirkung der erfindungsgemäßen Elastase-Inhibitoren auf die Verhinderung dieser Lyse zeigt.

### 2. In vivo-Wirkung der erfindungsgemäß verwendeten Elastase-Inhibitoren

Zur Bestimmung der Bedeutung von Leukozyten-Proteasen, insbesondere Elastase-Inhibitoren, im Rahmen der vorliegenden Erfindung, wurden zur Veranschaulichung der Blutstillung mittels Gewebekleber sowohl die Wirkung des erfindungsgemäßen Klebers in hyperfibrinolytischen Systemen als auch bei normaler fibrinolytischer Aktivität getestet und mit Klebern ohne Inhibitoren bzw. mit einem Kleber, welcher nur Aprotinin als Plasmin-Inhibitor beinhaltet, verglichen.

### 2.a) Hyperfibrinolyse

Anaesthesierte Kaninchen (2-3 kg) wurden heparinisiert (4000 E/kg). Eine halbe Stunde danach wurde ein Teil des rechten Leberlappens geklemmt und partiell distal von der Klemme reseziert. Blutungen aus größeren Gefäßen wurden mittels Elektrokoagulation gestoppt und die restliche diffuse Blutung durch Aufbringung von Gewebekleber (max. 4 ml) innerhalb von 200 Sekunden versiegelt. 10 Minuten danach wurde mit der Infusion von t-PA (700 E/kg/h) begonnen und für 2 Stunden das Ausmaß des "Rebleeding" bestimmt, indem die Gewichtszunahme von vorgewogenen Tupfern gemessen wurde.

Dabei wurden 3 verschiedene Gewebekleber getestet.
a) Gewebekleber (STIM3) mit Aprotinin (3000 E/ml) als Negativkontrolle
b) Gewebekleber (STIM3) ohne Aprotinin als Positivkontrolle
c) Gewebekleber (STIM3) ohne Aprotinin mit Eglin (10 µg/ml); erfindungsgemäßer Kleber

Diese Kleber wurden verblindet und mittels einer Duploject®-Spritze (Firma IMMUNO, Wien, AT) appliziert.

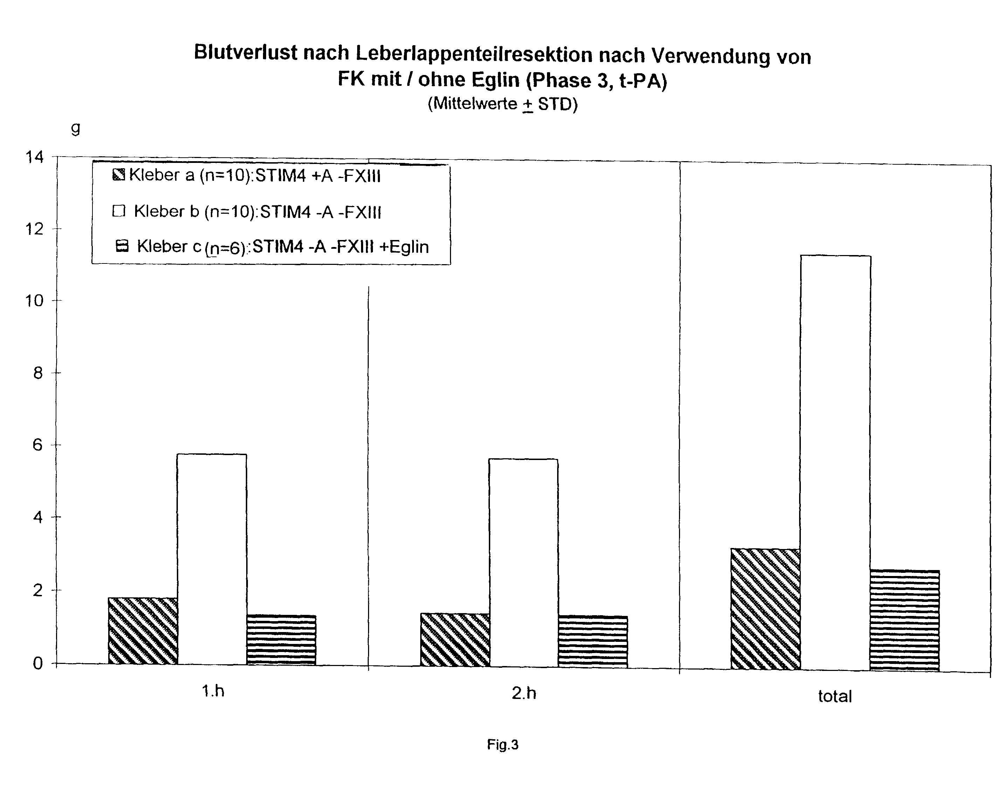

Die erhaltenen Ergebnisse sind in Figur 3 dargestellt.

### 2.b) Normale fibrinolytische Aktivität

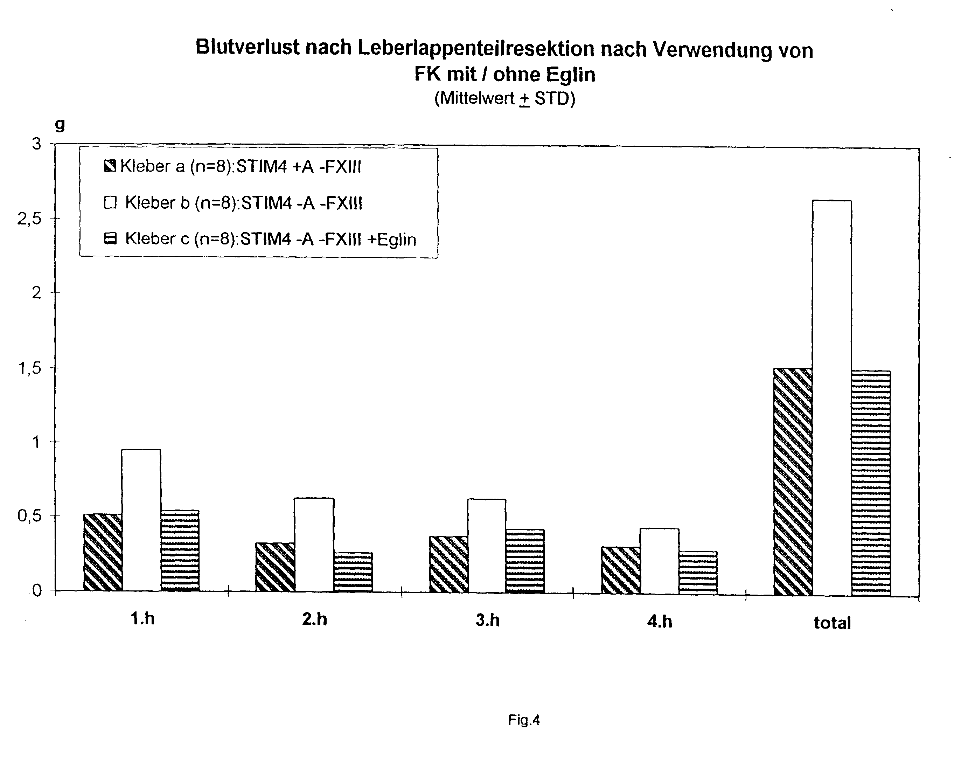

Zusätzlich zum Hyperfibrinolysemodell wurden auch noch gleiche Versuche ohne t-PA-Infusion unternommen, jedoch mit verlängerter Beobachtungszeit von 4 Stunden. Die erhaltenen Ergebnisse sind in Figur 4 dargestellt.

Es zeigte sich, daß sowohl im Hyperfibrinolyse-Modell als auch bei der normalen Fibrinolyse ein gegenüber herkömmlichen Klebern verringertes "Rebleeding" ermöglicht wird, welches v.a. bei längeren Lysezeiten auch gegenüber Aprotinin verbesserte Eigenschaften aufweist.

Diese Ergebnisse belegen die ausgezeichneten Wirkungen der erfindungsgemäßen Gewebekleber, mit welchen eine frühzeitige Lyse des Fibrinklebers verhindert werden kann, womit erneute Blutungen ("Rebleedings") auch in Bereichen mit hoher fibrinolytischer Aktivität hintangehalten werden können.

## Patentansprüche

1. Gewebekleber auf Basis von Fibrinogen, **dadurch gekennzeichnet, daß** er einen zugesetzten Elastase-Inhibitor enthält.

2. Gewebekleber nach Anspruch 1, **dadurch gekennzeichnet, daß** der Elastase-Inhibitor ausgewählt ist aus der Gruppe Eglin, Elastase-α1-Proteinase-Inhibitor, α₁-Antiprotease, Leukozytenprotease-Inhibitor, Elafin oder Mischungen davon.

3. Gewebekleber nach Anspruch 2, **dadurch gekennzeichnet, daß** der Leukozytenprotease-Inhibitor als Leukozytenfraktion, insbesondere als eine von Granulozyten abgeleitete Fraktion, zur Verfügung gestellt wird.

4. Gewebekleber nach einem der Ansprüche 1 bis 3, **dadurch gekennzeichnet, daß** er ausschließlich aus menschlichen Proteinen zusammengesetzt ist.

5. Gewebekleber nach einem der Ansprüche 1 bis 4, **dadurch gekennzeichnet, daß** er ausschließlich aus menschlichen Blut- oder Plasmaproteinen zusammengesetzt ist.

6. Gewebekleber nach einem der Ansprüche 1 bis 5, **dadurch gekennzeichnet, daß** der Elastase-Inhibitor in einem Mengenverhältnis von 1:100 bis 1:150 000, bevorzugt 1:500 bis 1:110 000, bezogen auf mg Fibrinogen enthalten ist

7. Gewebekleber nach einem der Ansprüche 1 bis 6, **dadurch gekennzeichnet, daß** wenigstens 10⁻⁶ E Elastase-Inhibitor pro g Fibrinogen, vorzugsweise zwischen 10⁻³ und 10 E/g Fibrinogen enthalten sind.

8. Gewebekleber nach einem der Ansprüche 1 bis 7, **dadurch gekennzeichnet, daß** er Plasminogen in einer Menge von zumindest 0,0001 mg/mg Fibrinogen, vorzugsweise zumindest 0,001, am meisten bevorzugt mehr als 0,01 enthält.

9. Gewebekleber nach einem der Ansprüche 1 bis 7, **dadurch gekennzeichnet, daß** er kein Plasminogen enthält.

10. Gewebekleber nach einem der Ansprüche 1 bis 9, **dadurch gekennzeichnet, daß** er weiters einen Plasmin-Inhibitor oder einen Plasmin-Aktivator-Inhibitor, enthält, welcher vorzugsweise ausgewählt ist aus der Gruppe Aprotinin, α₂-Makroglobulin, α₁-Antitrypsin, ε-Aminocapronsäure, Tranexamsäure oder Mischungen davon.

11. Gewebekleber nach einem der Ansprüche 1 bis 10, **dadurch gekennzeichnet, daß** er ein Antibiotikum enthält, welches vorzugsweise ausgewählt ist aus der Gruppe Aminoglycoside, Betalactame, Polypeptide, Fosfomycin, Tetracycline oder Mischungen davon.

12. Gewebekleber nach einem der Ansprüche 1 bis 11, **dadurch gekennzeichnet, daß** er Faktor XIII, vorzugsweise in einer Menge von zumindest 0,001 E/mg Fibrinogen, besonders bevorzugt zumindest 0,1 E/mg enthält.

13. Gewebekleber nach einem der Ansprüche 1 bis 12, **dadurch gekennzeichnet, daß** er frei von kininogenen Proteinen ist.

14. Gewebekleber nach einem der Ansprüche 1 bis 13, **dadurch gekennzeichnet, daß** er in Kombination mit einer festen Oberfläche als Vlies vorliegt.

15. Gewebekleber nach Anspruch 14, **dadurch gekennzeichnet, daß** die feste Oberfläche eine Kollagen-, Gelatine-, oder Polysaccharid-Oberflache ist.

16. Gewebekleber nach einem der Ansprüche 1 bis 15, **dadurch gekennzeichnet, daß** er in einem Millieu mit hoher fibrinolytischer Aktivität für einen Zeitraum von wenigstens 10 Stunden, vorzugsweise wenigstens 15 Stunden, lysebeständig ist.

17. Gewebekleber nach einem der Ansprüche 1 bis 16, **dadurch gekennzeichnet, daß** er lyophilisiert ist.

18. Gewebekleber nach einem der Ansprüche 1 bis 16, **dadurch gekennzeichnet, daß** er in Lösung vorliegt.

19. Gewebekleber nach Anspruch 18, **dadurch gekennzeichnet, daß** die Lösung tiefgefroren ist.

20. Gewebekleber nach einem der Ansprüche 1 bis 19, **dadurch gekennzeichnet, daß** er in virusinaktivierter Form vorliegt.

21. Gewebekleber nach einem der Ansprüche 1 bis 20, **dadurch gekennzeichnet, daß** der Elastase-Inhibitor rekombinanten Ursprungs ist.

22. Gewebekleber-System, **dadurch gekennzeichnet, daß** es als eine Komponente einen Gewebekleber nach einem der Ansprüche 1 bis 21 umfaßt.

23. Gewebekleber-System nach Anspruch 22, **dadurch gekennzeichnet, daß** es weiters eine Komponente, die Thrombin und gegebenenfalls Calcium enthält, umfaßt.

24. Gewebekleber-System, **dadurch gekennzeichnet, daß** es eine Fibrinogen-Komponente und eine Komponente umfaßt, die einen Elastase-Inhibitor enthält.

25. Gewebekleber-System nach Anspruch 24, **dadurch gekennzeichnet, daß** die Komponente, die einen Elastase-Inhibitor enthält, Thrombin enthält.

26. Gewebekleber-System nach einem der Ansprüche 22 bis 25, **dadurch gekennzeichnet, daß** es weiters eine Applikationsvorrichtung für die System-Komponente(n) umfaßt, insbesondere ein Doppelspritzen-System.

27. Verwendung eines Gewebeklebers nach einem der Ansprüche 1 bis 19 zur Herstellung einer Präparation zur Anwendung in Bereichen mit hoher fibrinolytischer Aktivität, insbesondere in der Urologie.

28. Verwendung eines Gewebekleber-Systems nach einem der Ansprüche 22 bis 26 zur Herstellung einer Applikationsvorrichtung zur Anwendung in Bereichen mit hoher fibrinolytischer Aktivität, insbesondere in der Urologie.

## Claims

1. A fibrinogen-based tissue adhesive, **characterised in that** it comprises an admixed elastase inhibitor.

2. A tissue adhesive according to claim 1, **characterised in that** the elastase inhibitor is selected from the group of eglin, elastase-α₁-proteinase inhibitor, α₁-antiprotease, leukocyte protease inhibitor, elafin, or mixtures thereof.

3. A tissue adhesive according to claim 2, **characterised in that** the leukocyte protease inhibitor is provided as a leukocyte fraction, in particular a granulocyte-derived fraction.

4. A tissue adhesive according to any one of claims 1 to 3, **characterised in that** it is composed exclusively of human proteins.

5. A tissue adhesive according to any one of claims 1 to 4, **characterised in that** it is composed exclusively of human blood- or plasma proteins.

6. A tissue adhesive according to any one of claims 1 to 5, **characterised in that** the elastase inhibitor is contained in an amount ratio of from 1:100 to 1:150,000, preferably 1:500 to 1:110,000, based on mg of fibrinogen.

7. A tissue adhesive according to any one of claims 1 to 6, **characterised in that** at least 10⁻⁶ U of elastase inhibitor are contained per g of fibrinogen, preferably between 10⁻³ and 10 U/g of fibrinogen.

8. A tissue adhesive according to any one of claims 1 to 7, **characterised in that** it contains plasminogen in an amount of at least 0.0001 mg/mg of fibrinogen, preferably at least 0.001, most preferred more than 0.01.

9. A tissue adhesive according to any one of claims 1 to 7, **characterised in that** it does not contain any plasminogen.

10. A tissue adhesive according to any one of claims 1 to 9, **characterised in that** it further comprises a plasmin inhibitor or a plasmin activator inhibitor which preferably is selected from the group of aprotinin, α₂-macroglobulin, α₁-antitrypsin, ε-amino-caproic acid, tranexamic acid, or mixtures thereof.

11. A tissue adhesive according to any one of claims 1 to 10, **characterised in that** it comprises an antibiotic which preferably is selected from the group of aminoglycosides, betalactams, polypeptides, phosphomycin, tetracyclines or mixtures thereof.

12. A tissue adhesive according to any one of claims 1 to 11, **characterised in that** it comprises factor XIII, preferably in an amount of at least 0.001 U/mg of fibrinogen, particularly preferred at least 0.1 U/mg.

13. A tissue adhesive according to any one of claims 1 to 12, **characterised in that** it is free from kininogenic proteins.

14. A tissue adhesive according to any one of claims 1 to 13, **characterised in that** it is present in combination with a solid surface as a fleece.

15. A tissue adhesive according to claim 14, **characterised in that** the solid surface is a collagen, gelatin or polysaccharide surface.

16. A tissue adhesive according to any one of claims 1 to 15, **characterised in that** in an environment of high fibrinolytic activity it is resistant to lysis for a period of time of at least 10 hours, preferably at least 15 hours.

17. A tissue adhesive according to any one of claims 1 to 16, **characterised in that** it is lyophilized.

18. A tissue adhesive according to any one of claims 1 to 16, **characterised in that** it is present in solution.

19. A tissue adhesive according to claim 18, **characterised in that** the solution is deep-frozen.

20. A tissue adhesive according to any one of claims 1 to 19, **characterised in that** it is present in virus-inactivated form.

21. A tissue adhesive according to any one of claims 1 to 20, **characterised in that** the elastase inhibitor is of recombinant origin.

22. A tissue adhesive system, **characterised in that** it comprises a tissue adhesive according to any one of claims 1 to 21 as one component thereof.

23. A tissue adhesive system according to claim 22, **characterised in that** it further comprises a component which comprises thrombin and, optionally, calcium.

24. A tissue adhesive system, **characterised in that** it comprises a fibrinogen component and a component which comprises an elastase inhibitor.

25. A tissue adhesive system according to claim 24, **characterised in that** the component which comprises an elastase inhibitor comprises thrombin.

26. A tissue adhesive system according to any one of claims 22 to 25, **characterised in that** it further comprises an application device for the component(s) of the system, in particular a double-syringe system.

27. The use of a tissue adhesive according to any one of claims 1 to 19 for producing a preparation to be applied in fields with high fibrinolytic activity, in particular in urology.

28. The use of a tissue adhesive system according to any one of claims 22 to 26 for producing an application device to be employed in fields with high fibrinolytic activity, in particular in urology.

## Revendications

1. Adhésif tissulaire à base de fibrinogène, **caractérisé en ce qu'**il contient un inhibiteur d'élastase ajouté.

2. Adhésif tissulaire selon la revendication 1, **caractérisé en ce que** l'inhibiteur d'élastase est choisi parmi le groupe de l'égline, l'inhibiteur de protéinase élastase-α1, l'antiprotéase α1, l'inhibiteur de protéase de leucocyte, l'élafine ou leurs mélanges.

3. Adhésif tissulaire selon la revendication 2, **caractérisé en ce que** l'inhibiteur de protéase de leucocyte est mis à disposition sous la forme d'une fraction de leucocyte, en particulier sous la forme d'une fraction dérivée des granulocytes.

4. Adhésif tissulaire selon l'une quelconque des revendications 1 à 3, **caractérisé en ce qu'**il se compose exclusivement de protéines humaines.

5. Adhésif tissulaire selon l'une quelconque des revendications 1 à 4, **caractérisé en ce qu'**il se compose exclusivement de protéines sanguines ou plasmiques humaines.

6. Adhésif tissulaire selon l'une quelconque des revendications 1 à 5, **caractérisé en ce que** l'inhibiteur d'élastase est présent en un rapport quantitatif allant de 1:100 à 1:150 000, de préférence de 1:500 à 1:110 000, sur base des mg de fibrinogène.

7. Adhésif tissulaire selon l'une quelconque des revendications 1 à 6, **caractérisé en ce qu'**au moins 10⁻⁶ U d'inhibiteur d'élastase sont présents par gramme de fibrinogène, de préférence de 10⁻³ à 10 U/g fibrinogène.

8. Adhésif tissulaire selon l'une quelconque des revendications 1 à 7, **caractérisé en ce qu'**il contient le plasminogène en une quantité d'au moins 0,0001 mg/mg fibrinogène, de préférence au moins 0,001, de manière la plus préférée plus de 0,01.

9. Adhésif tissulaire selon l'une quelconque des revendications 1 à 7, **caractérisé en ce qu'**il ne contient pas de plasminogène.

10. Adhésif tissulaire selon l'une quelconque des revendications 1 à 9, **caractérisé en ce qu'**il contient en outre, un inhibiteur de plasmine ou un inhibiteur d'activateur de plasmine, qui est choisi de préférence, parmi le groupe de l'aprotinine, l'α₂-macroglobuline, l'α₁-antitrypsine, l'acide ε-aminocaproïque, l'acide tranexamique ou leurs mélanges.

11. Adhésif tissulaire selon l'une quelconque des revendications 1 à 10, **caractérisé en ce qu'**il contient un antibiotique, qui est choisi de préférence, parmi le groupe des aminoglycosides, bêtalactames, polypeptides, la fosfomycine, les tétracyclines ou leurs mélanges.

12. Adhésif tissulaire selon l'une quelconque des revendications 1 à 11, **caractérisé en ce qu'**il contient le facteur XIII, de préférence en une quantité d'au moins 0,001 U/mg de fibrinogène, de manière particulièrement préférée, au moins 0,1 U/mg.

13. Adhésif tissulaire selon l'une quelconque des revendications 1 à 12, **caractérisé en ce qu'**il est exempt de protéines kininogènes.

14. Adhésif tissulaire selon l'une quelconque des revendications 1 à 13, **caractérisé en ce qu'**il se présente en combinaison avec une surface solide comme un voile.

15. Adhésif tissulaire selon la revendication 14, **caractérisé en ce que** la surface solide est une surface en collagène, gélatine ou polysaccharide.

16. Adhésif tissulaire selon l'une quelconque des revendications 1 à 15, **caractérisé en ce qu'**il est stable à la lyse dans un milieu avec une activité fibrinolytique élevée pendant une période d'au moins 10 heures, de préférence au moins 15 heures.

17. Adhésif tissulaire selon l'une quelconque des revendications 1 à 16, **caractérisé en ce qu'**il est lyophilisé.

18. Adhésif tissulaire selon l'une quelconque des revendications 1 à 16, **caractérisé en ce qu'**il se présente comme une solution.

19. Adhésif tissulaire selon la revendication 18, **caractérisé en ce que** la solution est congelée.

20. Adhésif tissulaire selon l'une quelconque des revendications 1 à 19, **caractérisé en ce qu'**il est présent sous une forme inactivée au niveau des virus.

21. Adhésif tissulaire selon l'une quelconque des revendications 1 à 20, **caractérisé en ce que** l'inhibiteur d'élastase est d'origine recombinée.

22. Système d'adhésif tissulaire, **caractérisé en ce qu'**il comprend comme composant, un adhésif tissulaire selon l'une quelconque des revendications 1 à 21.

23. Système d'adhésif tissulaire selon la revendication 22, **caractérisé en ce qu'**il comprend par ailleurs, un composant qui contient de la thrombine et le cas échéant, du calcium.

24. Système d'adhésif tissulaire, **caractérisé en ce qu'**il contient un composant fibrinogène et un composant qui contient un inhibiteur d'élastase.

25. Système d'adhésif tissulaire selon la revendication 24, **caractérisé en ce que** le composant qui contient un inhibiteur d'élastase, contient de la thrombine.

26. Système d'adhésif tissulaire selon l'une quelconque des revendications 22 à 25, **caractérisé en ce qu'**il comprend par ailleurs, un dispositif d'application pour le ou les composants du système, en particulier un système de pulvérisation.

27. Utilisation d'un adhésif tissulaire selon l'une quelconque des revendications 1 à 19, pour la préparation d'une préparation à utiliser dans des zones à activité fibrinolytique élevée, en particulier en urologie.

28. Utilisation d'un système d'adhésif tissulaire selon l'une quelconque des revendications 22 à 26, pour la préparation d'un dispositif d'application à utiliser dans des zones à activité fibrinolytique élevée, en particulier en urologie.
